# EUROPEAN PATENT SPECIFICATION

(11) **EP 1 387 691 B1**
(45) Date of publication and mention of the grant of the patent: **16.08.2006**
(21) Application number: 02723117.4
(22) Date of filing: 07.02.2002
(51) Int. Cl.: A61K 38/48

(54) **BLOOD COAGULATION FACTOR XIII FOR TREATING PLATELET DISORDERS**
BLUTGERINNUNGSFAKTOR XIII ZUR BEHANDLUNG VON PLÄTTCHENFUNKTIONSSTÖRUNGEN
FACTEUR XIII DE COAGULATION SANGUINE PERMETTANT DE TRAITER DES PATHOLOGIES PLAQUETTAIRES

(30) Priority: 21.02.2001 US 270470 P
(43) Date of publication of application: 11.02.2004
(73) Proprietor: ZymoGenetics, Inc., Seattle, WA 98102 (US)
(72) Inventor: BISHOP, Paul, D., Fall City, WA 98024 (US)
(74) Representative: MacLean, Martin Robert
(86) International application number: PCT/US2002/003770
(87) International publication number: WO 2002/067981

(56) References cited:
- EP-A- 0 225 160
- WO-A-93/12813
- WO-A-94/11022
- WO-A-96/28153
- US-A- 5 612 456
- RASCHE, H. ET AL.: "Blutgerinnungsfactor-XIII-Substitution bei akuter Leukämie: Ergebnisse einer randomisierten und kontrollierten Studie" DTSCH. MED. WSCHR., vol. 107, no. 49, 1982, pages 1882-1886, XP001093802

## Description

### BACKGROUND OF THE INVENTION

Platelet disorders may cause defective formation of hemostatic plugs and bleeding because of decreased platelet numbers (thrombocytopenia) or because of decreased function despite adequate platelet numbers. Thrombocytopenia is a condition in which an individual has a platelet count below the normal range of 250,000 - 500,000 µL. Thrombocytopenia may stem from failed platelet production, splenic sequestration of platelets, increased platelet destruction, or use, or dilution. Regardless of cause, severe thrombocytopenia and platelet dysfunction results in a typical pattern of bleeding: multiple petechiae in the skin, often most evident on the lower legs, scattered small ecchymoses at sites of minor trauma, mucosal bleeding (epistaxis, bleeding in the gastrointestinal tract (GI) and genitalurinary tract (GU), vaginal bleeding). Heavy GI bleeding and bleeding into the central nervous system (CNS) may be life threatening. Thus, there is a need for a treatment to inhibit bleeding caused by failed platelet production, increased platelet destruction or use.

Rasche, H. *et al,* DTSCH.MED.WSCHR., Vol.107, No.49, (1982) pages 1882-1886 describes the use of factor XIII substitution in acute leukaemia.

WO 94/11022 describes the use of factor XIII to inhibit post-operative haemorrhaging.

WO 93/12813 describes the use of factor XIII to reduce blood loss during surgery.

EP 0225160 describes the use of factor VIIa to treat bleeding disorders such as thrombocytopenia and other platelet disorders.

WO96/28153 describes the use of Ascochyta metabolites for reducing blood loss.

US 5 612456 describes methods for purifying factor XIII.

Havemann K., *et al* KLIN.WSCHR., Vol. 55, pages 801-809, describes factor XIII deficiency and substitution therapy with factor XIII.

The present invention provides use of factor XIII in the manufacture of a medicament for providing symptomatic relief of chemically induced or metabolically induced thrombocytopaenia.

The present invention also provides use of factor XIII in the manufacture of a medicament for treating platelet dysfunction.

Examples of conditions that can lead to failed platelet production are leukaemia, aplastic anemia, paroxysmal nocturnal hemoglobinuria, alcohol induced thrombocytopenia, thrombocytopenia in megaloblastic anemias, human immunodeficiency virus (HIV)-associated thrombocytopenia, idiopathic thrombocytopenic purpura, and myelodysplaxtic syndromes. Sequestration of platelets in enlarged spleens can also cause thrombocytopenia. This is can be caused by cirrhosis with congestive splenomegaly, myelofibrosis with myeloid metaplasia, and Gaucher's disease. Pathologic destruction of platelets may also result in thrombocytopenia. This is very often caused by platelets being coated by antibodies and then being removed by mononuclear phagocytes induced by idiopathic thrombocytopenic purpura, HIV-associated thrombocytopenia, drug induced thrombocytopenia and neonatal alloimmune thrombocytopenia. Platelet destruction can also be induced by thrombin-induced platelet damage as occurs in states with intravascular coagulation such as in complications of obstetrics, metastatic malignancy, septicemia and traumatic brain damage. Platelet destruction can also be caused by acute vascular abnormalities as is often found in thrombotic thrombocytopenic purpura-hemolytic-uremic syndrome, thrombocytopenia in adult respiratory distress syndrome and severe infections with septicemia. Thrombocytopenia can also be caused by such agents as quinidine, chemotherapy drugs, quinine, heparin, radiation, nonsteroid anti-inflammatory drugs (NSAIDs) such as aspirin, ibuprofen and naproxen.

The use according to the present invention provides symptomatic relief of the thrombocytopenia or platelet dysfunction by administering factor XIII. The administration of factor XIII can be applied prophylactically or at the time of a bleeding episode.
Factor XIII, also known as fibrin-stabilizing factor, circulates in the plasma at a concentration of 20 ?g/ml. The protein exists in plasma as a tetramer comprised of two A subunits and two B subunits. Each subunit has a molecular weight of 83,000 Da, and the complete protein has a molecular weight of approximately 330,000 Da. Factor XIII catalyzes the cross-linkage between the γ-glutamyl and ε-lysyl groups of different fibrin strands. The catalytic activity of factor XIII resides in the A subunits. The B subunits act as carriers for the A subunits in plasma factor XIII. Recombinant factor XIII can be produced according to the process described in European Patent No. 0 268 772 B1. The level of factor XIII in the plasma can be increased by administering a factor XIII concentrate derived from human placenta called FIBROGAMMIN® (Aventis Corp.) or by administration of recombinant factor XIII. When recombinant factor XIII is used, only the 'A₂'homodimer is generally administered without the 'B₂' subunit.

Administration of factor XIII to a subject is generally done intravenously. When administering therapeutic proteins by injection, the administration may be by continuous infusion or by single or multiple boluses. A pharmaceutical composition comprising factor XIII can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. A suitable pharmaceutical composition of factor XIII will contain 1mM EDTA, 10mM Glycine, 2% sucrose in water. An alternative formulation will be a factor XIII composition containing 20 mM histidine, 3% wt/volume sucrose, 2 mM glycine and .01% wt/vol. polysorbate, pH 8. The concentration of factor XIII should preferably be 1 - 10 mg/mL, more preferably about 5 mg/mL.

Other suitable carriers are well known to those in the art. See, for example, Gennaro (ed.), *Remington's Pharmaceutical Sciences,* 19th Edition (Mack Publishing Company 1995).

### Administration of Factor XIII

Factor XIII can be administered intravenously, intramuscularly or subcutaneously to treat platelet dysfunction or thrombocytopenia caused by metabolic disease, chemical agents or radiation. The levels of factor XIII in an individual can be determined by assays well known in the art such as the BERICHROM® F XIII assay (Dade Behring Marburgh GmbH, Marburg, Germany). The normal adult has an average of about 45 ml of plasma per kg of body weight. Each liter of blood has 1000 units (U) of factor XIII. The amount of factor XIII administered should be enough to bring an individual's level of factor XIII in the plasma to at least 100% of normal plasma or preferably 1-5% above normal. A dose of 45 U/kg would raise the level of factor XIII by about 1% compared to normal. One unit of factor XIII is about 10 µg of recombinant factor XIII, which contains only the dimerized A subunit. Thus, to raise the level of factor XIII by 1%, one would administer about 4.5 µg of the A2 subunit per kilogram weight of the individual. So to raise the level 30% of normal, one would administer 13.5 U/kg. For a 75 kg individual this would be about 1,012.5 U. Some patients may have consumptive coagulopathies that involve factor XIII losses. In such cases, a higher dosing (*e.g*., 1-2U/kg-%) or multiple dosing of factor XIII (*e.g*., 1-2U/kg-%-day) may be required.

## Claims

1. Use of factor XIII in the manufacture of a medicament for providing symptomatic relief of chemically-induced or metabolically induced thrombocytopaenia.

2. Use according to Claim 1, wherein the thrombocytopaenia is caused by a condition selected from the group consisting of leukaemia, aplastic anaemia, megaloblastic anaemia, human immunodeficiency virus-associated thrombocytopaenia, idiopathic thrombocytopaenia purpura and myelodysplastic syndromes.

3. Use according to claim 1, wherein the thrombocytopaenia is a condition selected from the group consisting of cirrhosis with congestive splenomegaly, myelofibrosis with myeloid metaplasia and Gaucher's disease.

4. Use according to claim 1, wherein the thrombocytopaenia is caused by alcohol, quinidine, chemotherapeutic drugs, quinine, heparin, radiation and nonsteroidal anti-inflammatory drugs.

5. Use of factor XIII in the manufacture of a medicament for treating platelet dysfunction.

6. Use according to claims 1-5, wherein the factor XIII is administered during a bleeding episode.

7. Use according to any previous claim, wherein the factor XIII is administered in an amount sufficient to raise factor XIII levels in a patient's plasma to a level of between 1% and 5% above normal.

## Patentansprüche

1. Verwendung von Faktor XIII bei der Herstellung eines Arzneimittels zum Bereitstellen einer Erleichterung der Symptome chemisch induzierter oder metabolisch induzierter Thrombozytopenie.

2. Verwendung nach Anspruch 1, wobei die Thrombozytopenie von einem Zustand verursacht wird, der aus der Gruppe bestehend aus Leukämie, aplastischer Anämie, megaloblastärer Anämie, mit dem humanen Immunschwåchevirus assoziierter Thrombozytopenie, idiopathischer thrombozytopenischer Purpura und myelodysplastischen Syndromen ausgewählt ist.

3. Verwendung nach Anspruch 1, wobei es sich bei der Thrombozytopenie um einen Zustand handelt, der aus der Gruppe bestehend aus Zirrhose mit Banti-Syndrom, Myelofibrose mit myeloischer Metaplasie und Gaucher'-Erkrankung ausgewählt ist.

4. Verwendung nach Anspruch 1, wobei die Thrompozytopenie von Alkohol, Chinidin, Chemotherapeutika, Chinin, Heparin, Strahlung und nicht-steroidalen Antirheumatika (NSAIDs) verursacht wird.

5. Verwendung von Faktor XIII bei der Herstellung eines Arzneimittels zum Behandeln von Thrombozytendysfunktion.

6. Verwendung nach den Ansprüchen 1 - 5, wobei der Faktor XIII während einer Blutungsepisode verabreicht wird.

7. Verwendung nach einem der vorhergehenden Ansprüche, wobei der Faktor XIII in einer Menge verabreicht wird, die dazu ausreicht, die Paktor-XIII-Spiegel im Plasma eines Patienten auf einen Spiegel von 1% bis 5 % über dem Normalwert anzuheben.

## Revendications

1. Utilisation du facteur XIII dans la fabrication d'un médicament permettant d'apporter un soulagement symptomatique de la thrombocytopénie induite chimiquement ou induite métaboliquement.

2. Utilisation selon la revendication 1, dans laquelle la thrombocytopénie est provoquée par une maladie choisie dans le groupe constitué par la leucémie, l'anémie aplastique, l'anémie mégaloblastique, la thrombocytopénie associée au virus de l'immunodéficience humaine, le purpura thrombocytopénique idiopathique et les syndromes myélodysplastiques.

3. Utilisation selon la revendication 1, dans laquelle la thrombocytopénie est une maladie choisie dans le groupe constitué par la cirrhose avec splénomégalie congestive, la myélofibrose avec métaplasie myéloïde et la maladie de Gaucher.

4. Utilisation selon la revendication 1, dans laquelle la thrombocytopénie est provoquée par l'alcool, la quinidine, les médicaments chimiothérapeutiques, la quinine, l'héparine, les rayons et les drogues antiinflammatoires non stéroïdes.

5. Utilisation du facteur XIII dans la fabrication d'un médicament permettant de traiter une pathologie plaquettaire.

6. Utilisation selon les revendications 1 à 5, dans laquelle le facteur XIII est administré pendant un épisode hémorragique.

7. Utilisation selon l'une quelconque des revendications précédentes, dans laquelle le facteur XIII est administré en quantité suffisante pour élever les niveaux de facteur XIII dans le plasma d'un patient à un niveau se situant entre 1 % et 5 % au-dessus du niveau normal.
